# EUROPEAN PATENT APPLICATION

(11) **EP 3 795 163 A1**
(43) Date of publication of application: **24.03.2021**
(21) Application number: 19198998.7
(22) Date of filing: 23.09.2019
(51) Int. Cl.: A61K 36/185, A61K 31/07, A61P 17/06

(54) **A TOPICAL PLANT EXTRACT FORMULATION COMPRISING URTICA DIOICA AND VITAMIN A**

(71) Applicant: The Ceutics Company GesmbH, 1010 Vienna (AT)
(72) Inventor: FRITZ, Stefan Ulrich, 2371 Hinterbrühl (AT)
(74) Representative: Redl, Gerda

(57) **Abstract**

A topical formulation comprising a composition comprising a vitamin A compound and water extract of plant material of urtica dioica, and a pharmaceutically acceptable carrier.

## Description

### FIELD OF THE INVENTION

The present invention relates to the field of topical formulations comprising plant extracts, their use and production thereof. Specifically, the present invention relates to topical formulations comprising water extracts of plant materials, which are at least of urtica dioica.

### BACKGROUND OF THE INVENTION

The skin, in particular the epidermis, as a barrier organ of the human organism is subjected to external influences to a particular extent. Many intrinsic factors (e.g. genetic predisposition) and extrinsic factors (e.g. damage to the skin barrier) can lead to unaesthetic or painful symptoms.

Hand eczema (HE) is a multifactorial, often chronic disease, and then referred to as chronic hand eczema (CHE), which is commonly related to occupational or routine household activities. Exact etiology of the disease is difficult to determine. An estimated 2-10% of the worldwide population is likely to develop hand eczema at some point of time during life. It appears to be the most common occupational skin disease, comprising up to 80% or more of all occupational contact dermatitides. The most common type of hand eczema is irritant dermatitis, atopic hand eczema, and allergic contact dermatitis. Females are more commonly involved than males (2:1), possibly because of increased exposure to wet work and household chemicals. Irritant contact dermatitis (ICD) was found to be the cause of hand eczema in about 50% of the cases, whereas allergic contact dermatitis comprised about 15% of the cases. Due to the high incidence and prevalence of this pathology, it has enormous socioeconomic consequences and a massive impact on the patients' quality of life.

HE is a heterogenous dermatosis with limited therapeutic options due to a lack of international guidelines regarding classification of HE subtypes and treatment. To this day, the most common treatment of choice is topical corticosteroids or even oral steroids. The introduction of topical corticosteroids (TC) was considered to be the most significant landmark in the history of therapy of dermatological disorders. Gradually, a large number of newer TC molecules of varying potency were introduced into the market, rendering the therapy of various inflammatory cutaneous disorders more effective and less time consuming. However, the side-effects of topical corticosteroids, and even more so, oral steroids are numerous and often severe. The most common side effects include epidermal thinning, monomorphic acne, steroid atrophy, steroid rosacea, telangiectasia, perioral dermatitis, striae and other manifestations of a condition which has been collectively described as topical steroid damaged facies (TSDF) (Coondoo A. et al., 2014, Indian Dermatol Online, 5(4):416-425).

Unfortunately, treatment options beyond topical and short-term systemic corticosteroids are very limited. Systemic agents, such as for example cyclosporine and probiotics, as well as topical agents, such as for example delgocitinib and retapamulin, have demonstrated merely varying degrees of clinical efficacy, evaluated by subjective assessments and scoring indexes. The patients' dissatisfaction with these treatment options is high (Lee GR et al., Dermatologic Therapy, 2019 Jan 28;e12840).

EP2146730B1 discloses a hot-water extract of stinging nettle and its use as a topical analgesic.

Tretinoin, also known as all-trans retinoic acid (ATRA) is a form of vitamin A, a retinoid that has a long standing use in dermatology for indications such as melanoma, acne and anti-wrinkles and has been used as an oral treatment for hand eczema. However, oral retinoids, such as for example acitretin, alitretinoin and isotretinoin, as well as high concentrations of topical retinoids can harm an unborn child and therefore must not be used during pregnancy or by women planning to get pregnant. Tretinoin applied topically to the skin often causes side effects such as redness, swelling, blistering, peeling, or crusting of the skin and lower concentrations of tretinoin are ineffective.

In summary, treatment options for hand eczema and related skin diseases are very limited. Additional therapeutic options are therefore desperately needed to match the rising prevalence and burden of such diseases. There is a specific need for topical formulations that can be used to treat skin-related diseases or conditions with significantly reduced side effects. In particular in the cosmetics and pharmaceuticals industry, there is a constant need for agents for improving the complexion of human skin.

### SUMMARY OF THE INVENTION

It is the objective of the present invention to provide a topical formulation that is effective against skin-related diseases or conditions, specifically hand eczema. A particular objective is to treat chronic hand eczema with.

The objective is solved by the subject of the claims and as further described herein.

Surprisingly, it was found that a combination of vitamin A and water extract of urtica dioica as described herein can overcome the drawbacks of prior art treatment options.

According to the invention there is provided a topical formulation comprising a composition comprising or consisting of a water extract of plant material of urtica dioica and a vitamin A compound, and a pharmaceutically acceptable carrier.

Specifically, the plant extract composition comprised in the formulation described herein further comprises or consists of at least one extract of plant material of any one or more of punica granatum, hibiscus sabdariffa, or camellia sinensis. Specifically, the extract of plant material (herein also referred to as "plant extract") e.g., of punica granatum, hibiscus sabdariffa, or camellia sinensis, is a water extract. Specifically, all plant extracts used for the purpose described herein are water extracts.

According to a specific embodiment, the composition described herein comprises or consists of extracts of plant material of urtica dioica and punica granatum; or urtica dioica and hibiscus sabdariffa; or urtica dioica and camellia sinensis.

According to a further specific embodiment, the composition described herein comprises or consists of extracts of plant material of urtica dioica, punica granatum and camellia sinensis; or of urtica dioica, punica granatum and hibiscus sabdariffa; or of urtica dioica, hibiscus sabdariffa and camellia sinensis; or of urtica dioica, camellia sinensis and hibiscus sabdariffa.

According to yet a further specific embodiment, the composition described herein comprises or consists of extracts of plant material of urtica dioica, punica granatum, camellia sinensis and hibiscus sabdariffa.

Specifically, individually selected plant materials can be any one or more of peel, leaf, stem or flower. Preferably, the plant material of urtica dioica is leaf and/or stem material. Preferably, the plant material of camellia sinensis is leaf material. Preferably, the plant material of punica granatum is peel material. Preferably, the plant material of hibiscus sabdariffa is flower, specifically blossom, material.

According to a specific embodiment, the plant material of urtica dioica comprises or consists of leaf and/or stem material.

Specifically, the plant material of urtica dioica is extracted using water at a temperature ranging from 120°C to 140°C, preferably ranging from 130°C to 140°C, specifically about (+/-1°C) any one of 120, 121, 122, 123, 124, 125, 126, 127, 128, 129, 130, 131, 132, 133, 134, 135, 136, 137, 138, 139 or 140°C. Specifically, the plant material of urtica dioica is extracted using water and applying pressure greater than atmospheric pressure.

Specifically, the water extract of plant material, in particular the urtica dioica water extract, or the composition described herein comprises any one or more, or all of p-Coumaric acid, coffeoyl malate, coumarin and trans-ferulic acid.

Specifically, the composition described herein comprises a combination of a first mixture of p-Coumaric acid and caffeoyl malate and a second mixture of coumarin and trans-ferulic acid. Specifically, the first and the second mixture are present at a ratio of 0.5-2:1 (w/w), preferably 0.8-1.5:1. Specifically, the first and the second mixture are present at a ratio of about 0.5:1, 0.6:1, 0.7:1, 0.8:1, 0.9:1, 1:1, 1.1:1, 1.2:1, 1.3:1, 1.4:1, 1.5:1, 1.6:1, 1.7:1, 1.8:1, 1.9:1, or 2:1 (w/w).

According to a specific embodiment, each plant extract of urtica dioica, and optionally of any one or more of punica granatum, hibiscus sabdariffa, or camellia sinensis is comprised in the formulation provided herein as a dry water extract, e.g. obtainable upon drying the water-based extract to a remaining water content of less than any one of 5, 4, 3, 2, or 1 % (w/w).

Specifically, all plant extracts comprised in the formulation provided herein are at a concentration of 0.1 to 5 % (w dry substance/w), preferably 0.2 to 0.7 % (w dry substance/w).

According to a specific embodiment, the vitamin A compound described herein is vitamin A, a derivative of vitamin A or provitamin A, preferably selected from the group consisting of tretinoin, retinol, retinal, retinoic acid, palmitate, β-carotene, adapalene, tazarotene, lutein, and zeaxanthin. Preferably, when the vitamin A compound described herein is vitamin A, a derivative of vitamin A or provitamin A it is comprised in the formulation described herein at a concentration of 0.05 to 0.3 % (w/w), more specifically 0.05 to 0.15 % (w/w).

According to a preferred embodiment, the pharmaceutically acceptable carrier described herein comprises at least one emulsifier at a concentration of about 0.1 to 10 % (w/w). Specifically, the formulation described herein comprises at least one emulsifier at a concentration of any one of about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9 or 10.0 % (w/w).

Specifically, the pharmaceutically acceptable carrier comprises more than one emulsifier, comprising a total concentration of emulsifiers of 10 to 40 % (w/w), specifically about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 % (w/w). Specifically, the emulsifier is any one or more of glyceryl stearate, lanolin, carbomer, camellia sinensis leaf extract, olea europaea fruit oil, ricinus communis seed oil or propylene glycol. Preferably, the emulsifier comprises at least glyceryl stearate. Even more preferably, the emulsifier comprises glyceryl stearate, lanolin, carbomer, camellia sinensis leaf extract, olea europaea fruit oil, ricinus communis seed oil and propylene glycol.

According to a preferred embodiment, the pharmaceutically acceptable carrier described herein comprises at least one viscosity controlling agent at a concentration of about 0.1 to 10 % (w/w). Specifically, the formulation described herein comprises at least one viscosity controlling agent at a concentration of any one of about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9 or 10.0 % (w/w).

Specifically, the pharmaceutically acceptable carrier comprises more than one viscosity controlling agent, comprising a total concentration of viscosity controlling agents of 10 to 40 % (w/w), specifically about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 % (w/w). Preferably, the viscosity controlling agent is any one or more of betaine, lanolin, propylene glycol or carbomer. Preferably, the viscosity controlling agent comprises at least betaine and/or lanolin.

According to a preferred embodiment, the pharmaceutically acceptable carrier described herein comprises at least one skin conditioning agent at a concentration ranging from 0.1 to 10 % (w/w). Specifically, the formulation described herein comprises at least one skin conditioning agent at a concentration of any one of about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9 or 10.0 % (w/w).

Specifically, the pharmaceutically acceptable carrier comprises more than one skin conditioning agent, comprising a total concentration of skin conditioning agents of about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 % (w/w). Preferably the skin conditioning agent is any one or more of betaine, aloe vera extract, punica granatum, hibiscus sabdariffa, camellia sinensis, olea europaea fruit oil, carthamus tinctorius seed oil, ricinus communis seed oil, prunus amygdalus dulcis oil or allantoin.

Specifically, the formulation provided herein comprises further additives including any one or more of buffering agents, preservatives, antioxidants, anti-microbial agents, anti-fungal agents, UV absorbing agents, humectants, antistatics, and perfume.

Specifically, the formulation does not comprise a corticosteroid.

Specifically, the formulation provided herein is in the form of a cream, emulsion, gel, ointment, lotion, spray, solution, paste or tincture.

Further provided herein is a method of producing the formulation described herein, comprising providing a composition comprising said extract(s) and said vitamin A compound, and formulating with said pharmaceutically acceptable carrier.

Specifically, the method provided herein comprises the steps of:
i. providing the components of said composition, which are the vitamin A compound, the water extract of urtica dioica, and optionally at least one extract of plant material of any one or more of punica granatum, hibiscus sabdariffa, and camellia sinensis;
ii. mixing said components in an aqueous solution e.g., employing a solvent, specifically an aqueous solvent;
iii. adding at least one emulsifier;
iv. optionally further adding any one or more of viscosity controlling agents, skin conditioning agents, buffering agents, emollients, antimicrobial agents or preservatives,
wherein each of said steps ii., iii., and iv., are carried out at a temperature of 50-90°C, followed by allowing the mixture to cool (e.g. to less than any one of 50°C, 40°C, 30°, or to about room temperature) under stirring to form the topical formulation.

Specifically, step ii. is carried out at a higher temperature than step iii. and/or iv. According to a specific embodiment, step ii. is carried out at a temperature of about 80-90°C, step iii. is carried out at a temperature of about 70-90°C and step iv. is carried out at a temperature of about 50-70°C.

Specifically, any one or more of steps ii., iii., and iv., are carried out at a temperature which is independently selected from the following: about (meaning +/- 1°C) any one of 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89 or 90°C.

Preferably, step i. is carried out at a temperature ranging from 120°C to 140°C, specifically about 120, 121, 122, 123, 124, 125, 126, 127, 128, 129, 130, 131, 132, 133, 134, 135, 136, 137, 138, 139 or 140°C.

Further provided herein is the use of the formulation described herein in a cosmetic product, specifically selected from the group consisting of a skin cream, emulsion, ointment, gel, lotion, spray, solution, paste and tincture.

Further provided herein is the formulation described herein for use in the treatment of skin related diseases or conditions, preferably dermatitis, psoriasis or eczema. Specifically, the formulation described herein is provided for use in the treatment of hand eczema, specifically chronic hand eczema.

### FIGURES

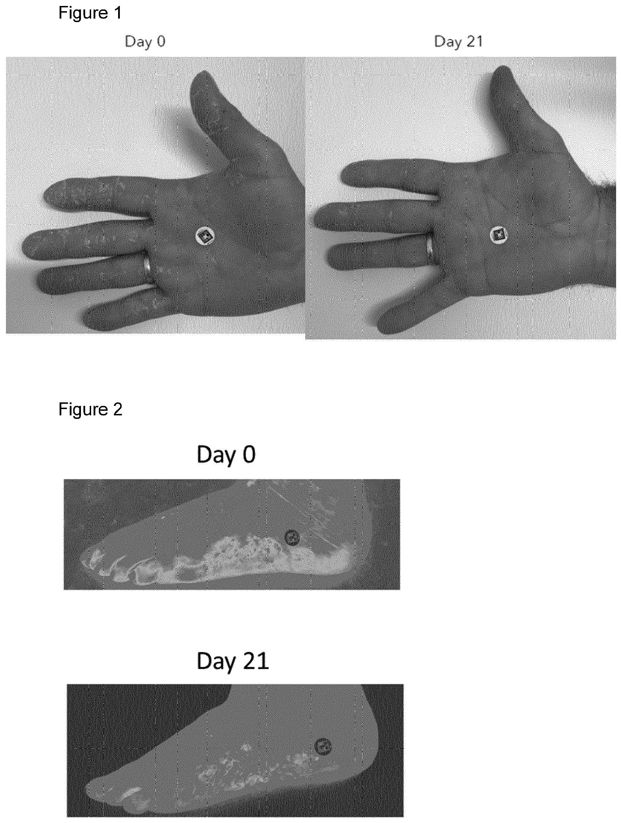

Figure 1 shows improvement of skin appearance in a patient suffering from chronic hand eczema after 3 weeks of treatment.
Figure 2 shows photo diagnosis showing reduced inflammation in a patient suffering from chronic hand eczema in the foot after 3 weeks of treatment.
Figure 3 summarizes results in 12 patients after 1 and 3 weeks of treatment.

### DETAILED DESCRIPTION

Unless indicated or defined otherwise, all terms used herein have their usual meaning in the art, which will be clear to the skilled person. Specific terms as used throughout the specification have the following meaning.

Unless indicated otherwise, the term "about" as used herein refers to the same value or a value differing by +/-5 % of the given value. The term "about" as used for °C temperature typically refers to +/- 1°C.

To provide a more concise description, some of the quantitative expressions given herein are not qualified with the term "about". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

As used herein, **"pharmaceutically acceptable"** means that the ingredients which the term describes are suitable for use in contact with tissues (e.g., the skin or hair) without undue toxicity, incompatibility, instability, irritation, allergic response, and the like.

The topical formulation provided herein is intended for application on the skin or outer surface of a subject, specifically for cosmetic or pharmaceutical purposes. As used herein, **"topical formulation"** refers to any pharmaceutically acceptable formulation, which may be applied to a subject's skin, hair, or nail, for example, by use of the hands or an applicator such as a wipe. Specifically, the topical formulation provided herein comprises vitamin A and extract of urtica dioica plant material. Surprisingly, the formulation described herein significantly improves symptoms associated with chronic inflammatory skin diseases, specifically chronic hand eczema. Specifically, treatment with the topical formulation provided herein of subjects suffering from chronic hand eczema, showed significant improvements in the appearance of the skin and reduction in pain and swelling.

As used herein, the term "subject" or "individual" or "patient" shall refer to a warm-blooded mammalian, particularly a human being e.g., an adult, child (including newborns). The subject may be a patient, specifically a patient in need of prophylaxis or treatment of a disease or disorder described herein.

The term "patient" includes mammalian, specifically human, subjects that receive either prophylactic or therapeutic treatment or are at risk of or diagnosed of a specific skin related disease or disorder, like, but not limited to, dermatitis, psoriasis or eczema.

The term **"treatment"** is meant to include both prophylactic and therapeutic treatment.

The terms **"skin related disease"** or **"skin related disorder"** as used herein, include all diseases or disorders in which skin is diseased or has a disorder, or in which skin is involved in an at least mediatory role. Specifically, skin related diseases or disorders, as referred to herein, are those, where treatment with a steroid, such as oral steroids or topical corticosteroids, is indicated or which are typically responsive to treatment with oral steroids or topical corticosteroids.

Specifically, skin related disease or disorder refers to an abnormal skin condition caused by inflammatory, allergic or autoimmune reactions. Typical symptoms of skin related diseases or disorders referred to herein include, for example, raised bumps that are red or white, a rash (which might be painful or itchy), scaly or rough skin, peeling skin, open sores or lesions, and dry, cracked skin. Specifically, the topical formulation referred to herein significantly reduces any one or more or all of these symptoms.

Inflammatory diseases of the skin include, for example, dermatitis, bacterial and viral skin inflammation, and acne. Dermatitis is an inflammation of the skin. Specifically, the term dermatitis includes atopic dermatitis, contact dermatitis, including irritant contact dermatitis and allergic contact dermatitis, allergic dermatitis, pruritic dermatitis, solar (UVB- induced) dermatitis and chemical-induced dermatitis.

Specifically, atopic dermatitis, also called eczema, is typically caused by a defect of stratum corneum, which is a protective wall located in the outermost part of the skin, caused by hereditary, environmental, or immunological factors and is exacerbated in arid climate. The symptoms of atopic dermatitis include for example severe pruritus (itch), xeroderma, eruption or oozing of the skin, boils and scale like skin (scaly skin). Scratching worsens symptoms and affected people have an increased risk of secondary skin infections.

Specifically, irritant contact dermatitis is an inflammation of the skin caused by contact of the skin to an irritant. An irritant is any agent capable of producing cell damage in any individual if applied for sufficient time and in sufficient concentration. Allergic processes are usually not involved, and dermatitis occurs without prior sensitization. Irritants cause damage by breaking or removing the protective layers of the upper epidermis. They denature keratin, remove lipids, and alter the water-holding capacity of the skin. This leads to damage of the underlying living cells of the epidermis. The severity of dermatitis produced by an irritant depends on the type of exposure, vehicle, and individual propensity. There is difference between the mechanism of acute and chronic irritant contact dermatitis. Chronic irritant contact dermatitis is typically due to disturbed barrier function and increased epidermal cell turn over while acute ICD is a type of inflammatory reaction.

Specifically, allergic contact dermatitis is an inflammation of the skin caused by a hypersensitivity reaction. Specifically, allergic contact dermatitis is a type IV hypersensitivity reaction, typically only affecting previously sensitized individuals. A common example of allergic contact dermatitis is the allergic reaction to plants, such as poison ivy, poison oak, and poison sumac. Another common example of allergic contact dermatitis is the reaction to insect bites or stings, such as mosquitos, bees and wasps. Symptoms of allergic contact dermatitis include reddening, swelling, itching and pain in the affected area.

Hand eczema and chronic hand eczema are types of dermatitis. Specifically, the most common types of hand eczema and chronic hand eczema are atopic hand eczema and contact dermatitis, specifically irritant contact dermatitis and allergic contact dermatitis.

Clinical patterns of hand eczema include for example pompholyx (vesicular eczema of palms and soles), dyshidrotic eczema, patchy vesiculosquamous eczema, hyperkeratotic palmar eczema (tylotic eczema), recurrent focal palmar peeling, discoid eczema (nummular eczema), wear and tear dermatitis (housewives dermatitis), ring eczema, fingertip eczema, apron eczema, and chronic acral dermatitis. Clinically, hand eczema is characterized by signs of erythema, vesicles, papules, scaling, fissures, hyperkeratosis, and symptoms of itch and pain. Chronic hand eczema is mostly localized to the hands of a subject. However, symptoms may also present themselves on other areas of the skin, such as the feet. Typically, hand eczema is considered chronic when symptoms are present for longer than 2, 3, 4, 5, 6, 7, 8, 9, 10, 11 or 12 months.

Allergic skin diseases or disorders typically include a condition of the skin caused by a hypersensitivity reaction. Specifically, a hypersensitivity reaction is an abnormal immune response to substances, which a healthy immune system does not react to or which are typically harmless to the organism. Typically, allergic reactions of the skin can include reddening, swelling, itchiness and pain. Specifically, such allergic reactions can cause or aid in the formation of eczema and/or urticaria, which is commonly known as hives, an outbreak of swollen, pale red bumps or plaques on the skin.

Autoimmune skin diseases or disorders typically arise from an abnormal immune response to an endogenous part or tissue of an organism, such as the skin. Examples of autoimmune diseases include psoriasis, lupus, dermatomyositis, and scleroderma.

Psoriasis is an inflammatory autoimmune disease characterized by patches of abnormal skin. These skin patches are typically red, itchy, and scaly. They may vary in severity from small and localized to complete body coverage. Injury to the skin can trigger psoriatic skin changes at that spot. There are five main types of psoriasis: plaque, guttate, inverse, pustular, and erythrodermic. Plaque psoriasis, also known as psoriasis vulgaris, makes up about 90% of cases.

The term skin related disease or disorder may further include UV-irritated skin, dry skin, detergent irritated skin (including irritation caused by enzymes and molecules used in washing detergents and sodium lauryl sulfate) and thinning skin (e.g. skin from the elderly and children).

As used herein, the term **"vitamin A compound"** refers to a molecule with Vitamin A activity, as determined in a RAR (retinoic acid receptor) or RXR (retinoid X receptor) agonistic activity assay.

There are different units which can be used to describe vitamin A, specifically the International Unit (IU), the Retinol Equivalent (RE), and the Retinol Activity Equivalent (RAE). One IU (also equal to 1 USP unit) is equivalent to 0.3 µg all-trans-retinol (molecular weight 286.6), 0.55 g retinyl palmitate (molecular weight 525), and 0.6 µg of beta-carotene. The current unit for human research, established by the US Institute of Medicine (IOM) in 2001, is the Retinol Activity Equivalent (RAE).

Specifically, vitamin A activity may be measured as retinol activity equivalent (RAE) according to IOM, wherein each µg RAE corresponds to 1 µg retinol, 2 µg of β-carotene in oil, 12 µg of dietary beta-carotene, or 24 µg of the other dietary provitamin-A carotenoids, specifically alpha-carotene, gamma-carotene, beta-cryptoxanthin (Institute of Medicine (US) Panel on Micronutrients. Dietary Reference Intakes for Vitamin A, Vitamin K, Arsenic, Boron, Chromium, Copper, Iodine, Iron, Manganese, Molybdenum, Nickel, Silicon, Vanadium, and Zinc. Washington (DC): National Academies Press (US); 2001. 4, Vitamin A.).

Specifically, vitamin A activity may also be measured as retinol equivalent (RE) according to National Research Council (NRC), wherein each µg RE corresponds to 1 µg retinol, 2 µg of β-carotene in oil, 6 µg of dietary beta-carotene, or 12 µg of the other dietary provitamin-A carotenoids, specifically alpha-carotene, gamma-carotene, beta-cryptoxanthin (National Research Council on *Diet and Health,* 1989).

Specifically, 1 RE or 1 RAE corresponds to 1µg of all-trans-retinol, all-trans-retinoic acid, tretinoin or isotretinoin.

The term "vitamin A" as used herein is understood to refer to a vitamin A compound as further described herein.

Specifically, the vitamin A compound used herein comprises or consists of one or more chemical or natural form of vitamin A, also called retinol, including precursors, derivatives of vitamin A and vitamers of vitamin A. Specific examples include one or more vitamers, e.g. retinoids, specifically any one or more of all-trans-retinol, all-trans-retinoic acid, tretinoin or isotretinoin.

The vitamin A compound as used herein may be any chemical form of vitamin A found in aqueous solutions, undissociated, in its free acid form or dissociated as an anion. The term as used herein includes all precursors or intermediates in the biotechnological vitamin A pathway.

Specifically, a vitamer of a particular vitamin is any of a number of chemical compounds, generally having a similar molecular structure, which shows vitamin activity.

Specifically, vitamers of vitamin A include retinoids and carotenoids.

Retinoids are a class of chemical compounds that are vitamers of vitamin A or are chemically related to it. Specifically, retinoids include beta carotene cleavage products also known as apocarotenoids, including but not limited to retinal, all-trans-retinoic acid, retinol, retinoic methoxide, retinyl acetate, retinyl esters, 4-keto-retinoids, 3 hydroxy-retinoids or combinations thereof. Specifically, long chain retinyl esters are hydrocarbon esters that consists of at least about 8, such as e.g. 9, 10, 12, 13, 15 or 20 carbon atoms and up to about 26, such as e.g. 25, 22, 21 or less carbon atoms, with preferably up to about 6 unsaturated bonds, such as e.g. 0, 1, 2, 4, 5, 6 unsaturated bonds. Long chain retinyl esters include but are not limited to linoleic acid, oleic acid or palmitic acid.

Specifically, all-trans-retinoic acid, or simpler, retinoic acid or tretinoin is a derivative of vitamin A, specifically it is a metabolite of vitamin A₁ (all-trans-retinol). Retinoic acid can be produced for example by two sequential oxidation steps that convert all-trans-retinol to retinaldehyde to all-trans-retinoic acid. Specifically, retinoic acid comprises the chemical formula C₂₀H₂₈O₂.

According to a specific embodiment, the composition described herein comprises a vitamin A compound which comprises or consists of one or more retinoids, in particular including or consisting of a retinoic acid, such as tretinoin, or a retinol or a retinol-like compound, such as isotretinoin, etretinate, and acitretin, or a combination of any of the foregoing.

Carotenoids comprise precursors of vitamin A which are also termed provitamins. Specifically, carotenoids are long, 40 carbon conjugated isoprenoid polyenes that are formed in nature by the ligation of two 20 carbon geranylgeranyl pyrophosphate molecules. These include but are not limited to phytoene, lycopene, and carotene, such as e.g. beta-carotene, which can be oxidized on the 4-keto position or 3-hydroxy position to yield canthaxanthin, zeaxanthin, or astaxanthin. Carotenoids that are particularly well suited for use in the present disclosure include Vitamin A precursors, such as beta-carotene, alpha-carotene, beta-cryptoxanthine, or mixtures thereof. Other carotenoids well suited for use in the present disclosure include lycopene, crocetin, lutein, or mixtures thereof.

According to a specific embodiment, the formulation described herein comprises vitamin A in the form of a retinoid at a concentration of 0,001- 0,5 % (w/w), preferably at a concentration of 0,05 - 0,3 % (w/w).

Specifically, the formulation described herein comprises vitamin A in the form of tretinoin at a concentration of 0,05 - 0,5 % (w/w), even more specifically at a concentration of at least any one of 0.05, 0.06, 0.07, 0.08, 0.09, 0.1, 0.11, 0.12, 0.13, 0.14, 0.15, 0.20, 0.25, 0.30, 0.35, 0.40, 0.45, or 0.50% (w/w), and preferably at about 0.1 % (w/w).

Specifically, the formulation described herein comprises vitamin A, preferably tretinoin, at a concentration of 0.05 to 0.5 mg/g of the formulation, preferably about 0.1, 0.2, 0.3 or 0.4 mg/g of the formulation.

Any vitamin A compound can be used as an alternative to tretinoin, in particular in about the same amount as herein described for tretinoin, or in about an amount that equals the respective RE or RAE as herein described for tretinoin.

The formulation described herein may be applied once or twice a day. Specifically, the formulation described herein is applied at least any one of 1, 2, 3, 4, or 5 times a day, or more.

Specifically, the vitamin A compound is used in a therapeutically or cosmetically effective amount.

According to a specific embodiment, the vitamin A compound is administered to a subject in a topical daily dose of up to 10 mg/day. Specifically, the vitamin A compound is administered in a topical daily dose of no more than any one of 5, 1 or 0.5 mg/day, or no more than any one of 100, 50, 10, 0.5, 0.1 or 0.05 µg/day. The amount of the vitamin A compound in the formulation or composition described herein is preferably such that the aforementioned doses are obtained. Such a content of vitamin A is especially suited in order to achieve the effects according to the invention, while applying low amounts of the composition to a subject's skin.

The term **"component"** with regard to a composition is herein understood as a part of a composition, which may include one or more further components and excipients. The composition comprised in the topical formulation described herein comprises at least the vitamin A component and the plant extract components, but may include further components, primarily phytogenic components and excipients.

The term **"water extract"** as used herein shall refer to the substances obtained upon extracting plant material(s) using an extractant which is pure water or an aqueous solution, including inorganic and/or organic substances in an aqueous solvent. Specifically, an aqueous solution is a liquid preparation that contains one or more chemical substances dissolved, i.e., molecularly dispersed, in a suitable solvent or mixture of mutually miscible solvents, wherein the predominant solvent is water. Specific examples of aqueous solutions are any of base solutions, acid solutions or salt solutions. Exemplary aqueous solutions may comprise hydrochloric acid, acetic acid, nitric acid, sodium hydroxide, potassium hydroxide, magnesium hydroxide, calcium hydroxide, sodium chloride, potassium chloride, and/or magnesium chloride.

The composition provided herein comprises at least one **extract of plant material,** also referred to as plant extract. A plant extract refers to a substance that has been removed from plant material. Plant extracts may be prepared by treating any plant material with a suitable extractant to obtain an extract comprising at least some components of the plant material from the plant material in aqueous solution or suspension. The plant material which is treated with extractant to provide a plant extract may be or may be derived from any part of a plant, for example, the leaves, bark, roots, flowers, seeds or fruits of the plant, preferably it is the leaves, stem or flower of the plant or the peel of the fruit or any combination thereof. In the preparation of plant extract, the plant components extracted from the plant material will be at least partially soluble or suspendable in the extractant. After the plant material has been treated with the extractant, the insoluble plant material may be left in the extractant, or the insoluble plant material may be separated from the extractant. The insoluble plant material may be separated from the extractant according to any appropriate method, for example, decantation, filtration or centrifugation. In preferred embodiments of the present invention, the insoluble plant material is separated from the extractant; the plant extract does not comprise insoluble components.

According to a preferred embodiment, the extractant is water, thus yielding a water extract of plant material.

According to a preferred embodiment, the one or more extracts of the plant materials as described herein are present in the formulation at a concentration of 0.1 to 5 % (w/w) each, preferably 0.2 to 0.7 % (w/w). Specifically, the plant extract(s) described herein are present in the formulation provided herein at a concentration of any one of about 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9 or 5.0 % (w/w) each.

Typically, the ratio w/w refers to weight dry substance per weight final formulation.

Methods for the preparation of plant extracts are well known to the person skilled in the art. Specifically, the plant extracts described herein can be obtained by decoction, digestion, percolation, soxlethtation, maceration or any other appropriate extraction method known to the person skilled in the art. Specifically, the plant extracts described herein are extracted using water or an aqueous solvent, specifically comprising or consisting of hot or cold water.

According to a specific embodiment, the plant extract is prepared by cold percolation extraction. Cold percolation extraction includes placing plant material in a perforated flask or vessel, and treating the plant material with cold extractant. The resulting plant extract will pass through the perforation, leaving the insoluble and/or insuspendable plant material in the flask or vessel. Suitable plant extract extractants include any which may be used to extract components from the plant material; that is, any extractant in which at least some of the components of the plant material are soluble or suspendable.

According to a further specific embodiment, the plant extract is prepared using hot water extraction techniques. Specifically, the plant extract is extracted under pressure at a temperature ranging from 120-140°C, preferably 130-140°C, most preferably at about 140°C. According to a specific example, sufficient pressure is applied to facilitate water extraction e.g. of more than 1 bar, in specific cases applying at least any one of 10, 15, 20, 25, 30, 35, 40, 45, or 50 bar, or even applying high pressure, such as up to any one of 200, 150, or 100 bar.

Preferably, water or an aqueous solvent is used for extraction in a quantity of 85 to 99 %, preferably 90 to 97 %, particularly 96 % (v/v), based on the total volume of the solution to be extracted comprising the aqueous solvent and the plant material. Furthermore, it is preferred that 1 to 10 %, preferably 5 to 7 %, particularly preferred 4 % (w/v), of plant material, preferably of urtica dioica is used, preferably in the form of dried and chopped leaves and stems. Preferably, the extraction is carried out over a period of 1 to 60 min, i.e. the composition to be extracted is kept at the selected extraction conditions (specific temperature and pressure) over a period of 1 to 60 min. The duration of the extraction typically has an effect on the depletion of volatile substances. Preference is given to 20-30 min extraction time. Preferably, the extract obtained is filtered in the cooling process at a temperature of at least about 80 to 90°C, preferably at least 85°C. If possible, the extracts are processed immediately, while still warm.

Specifically, the plant extract is an extractant-free extract. That is, a plant extract can be prepared according to the above methods, followed by removal of the extractant thereby obtaining a dry or non-aqueous extract.

*Urtica dioica* (stinging nettle) is a perennial plant belonging to the family of *Urticaceae,* genus *Urtica.* The composition provided herein comprises a water extract of urtica dioica, preferably a hot water extract. Specifically, flavonoids, tannins, volatile compounds, fatty acids, polysaccharides, isolectins, sterols, terpenes, protein, vitamins, and minerals are among the main chemical constituents of urtica dioica, flavonoids and caffeic acid derivatives contribute to the anti-inflammatory, antioxidant and analgesic activities of uritca leaves extracts.

Specifically, the urtica dioica extract provided herein comprises p-Coumaric acid, coffeoyl malate, coumarin and trans-ferulic acid, preferably as a combined extract comprising a first mixture of p-Coumaric acid and caffeoyl malate and a second mixture of coumarin and trans-ferulic acid. Specifically, the first mixture and the second mixture are present in the composition at a ratio of 0.5-2:1, preferably 0.8-1.5:1 (w/w, both dry substances).

According to a preferred embodiment, the plant extract of urtica dioica is produced under pressure at a temperature ranging from 120-140°C, preferably using the hot water extraction technique described above. Preferably, the urtica dioica extract is extracted from urtica dioica plant material using a temperature ranging from 120-140°C to generate an extract comprising at least about 0.7 to 0.8 µg/mL as a first mixture comprising p-coumaric acid and caffeoyl malate; and at least about 0.75 to 0.85 µg/mL as a second mixture comprising coumarin and trans-ferulic acid. Preferably, the topical formulation provided herein thus may comprise the first mixture of p-coumaric acid and caffeoyl malate and the second mixture of coumarin and trans-ferulic acid at a ratio of 0.8-1.1:1.

*Punica granatum,* commonly known as pomegranate, is a member of the monogeneric family, Punicaceae. Punica granatum and its chemical components possess various pharmacological and toxicological properties including antioxidant, anti-inflammatory (by inhibiting pro-inflammatory cytokines), anti-cancer and anti-angiogenesis activities. Extracts of punica granatum have been shown to elicit a broad spectrum of antioxidant activities and to exhibit scavenging activity against hydroxyl radicals and superoxide anions. The antioxidant action of punica granatum is observed, not only through its scavenging reactions, but also by its ability to form metal chelates.

According to a specific embodiment, the formulation provided herein comprises an extract of plant material of punica granatum.

Extracts of *hibiscus sabdariffa* (roselle or sour tea) showed antibacterial, antioxidant, nephro- and hepato-protective, renal/diuretic effect, effects on lipid metabolism (anti-cholesterol), anti-diabetic and anti-hypertensive effects among others. This might be linked to strong antioxidant activities, inhibition of α-glucosidase and α-amylase, inhibition of angiotensin-converting enzymes (ACE), and direct vaso-relaxant effect or calcium channel modulation. Phenolic acids (esp. protocatechuic acid), organic acid (hydroxycitric acid and hibiscus acid) and anthocyanins (delphinidin-3-sambubioside and cyanidin-3-sambubioside) are likely to contribute to the reported effects.

According to a specific embodiment, the formulation provided herein comprises an extract of plant material of hibiscus sabdariffa.

*Camellia sinensis* is an evergreen plant which can grow up to 25-30 feet and contains a significant amount of catechins. Catechins are a sub group of flavonoids and are thought to be responsible for the health benefits that have traditionally been attributed to the tea. Major catechins are epicatechin gallate (ECG), epicatechin, gallocatechin (GC), epigallocatechin (EGC) and epigallocatechin gallate (EGCG). GC, EGC and EGCG possess strong bactericidal as well as antibacterial activity.

The herbal composition described herein or the formulation described herein may be preserved by the addition of preservatives commonly used in the art such as sodium dehydroacetate, 2-hydroxybenzoic acid, 1-phenyl-1-propanol, Dermosoft® 700B (mixture of levulinic acid; sodium levulinate; glycerin and water), phenethyl alcohol or sodium benzoate.

According to a specific embodiment, the formulation provided herein comprises an extract of plant material of camellia sinensis.

According to a specific embodiment, the formulation provided herein comprises an extract of plant material of urtica dioica, an extract of plant material of punica granatum, an extract of plant material of hibiscus sabdariffa and an extract of plant material of camellia sinensis.

**Pharmaceutically acceptable carriers** generally include any and all suitable solvents, dispersion media, coatings, antibacterial and antifungal agents, isotonic and absorption delaying agents, and the like that are physiologically compatible the composition provided herein. As described herein, the formulation provided herein is applied topically to a subject's skin. Pharmaceutical carriers suitable for facilitating such means of administration are well-known in the art.

Pharmaceutically acceptable carriers generally include any and all suitable solvents, such as for example sterile water, saline, phosphate buffered saline, dextrose, glycerol, and the like, as well as combinations of any thereof.

In one embodiment, suitable pharmaceutically acceptable carriers include, but are not limited to, inert solid fillers or diluents and sterile aqueous or organic solutions (e.g., polyethylene glycol, propylene glycol, polyvinyl pyrrolidone, etc.). In certain such embodiments, suitable pharmaceutically acceptable excipients include, but are not limited to, water, salt solutions, alcohol, polyethylene glycols, gelatin, lactose, amylase, magnesium stearate, talc, silicic acid, viscous paraffin, hydroxymethylcellulose, polyvinylpyrrolidone, fillers, such as sugars (e.g., lactose, sucrose, mannitol, or sorbitol), and cellulose preparations (e.g., maize starch, wheat starch, rice starch, potato starch, gelatin, gum tragacanth, methyl cellulose, hydroxypropylmethylcellulose, sodium carboxymethylcellulose, and/or polyvinylpyrrolidone PVP).

Additional pharmaceutically acceptable carriers are known in the art and described in, *e.g.,* REMINGTON'S PHARMACEUTICAL SCIENCES.

According to a specific embodiment, pharmaceutically acceptable carriers and other additives, particularly other than water, can generally be included in the formulation described herein in quantities of 1 to 95 % (w/w), preferably 5 to 70 % (w/w), more preferably 5 to 50 % (w/w), in each case based on the total weight of formulation. The amounts of cosmetic or dermatological auxiliary agents and additives and perfume to be used in each case can easily be determined by the person skilled in the art by simple trials, depending on the nature of the particular product. According to a further specific embodiment, the formulation described herein preferably contains water in a quantity of up to 98 % (w/w), preferably 10 to 95 % (w/w), more preferably 40 to 90 % (w/w), in each case based on the total weight of the formulation.

Topical formulations described herein can be solutions, emulsions or suspensions and can include excipients such as suspending agents, solubilizers, surfactants, preservatives, and chelating agents.

The topical formulation provided herein may be one of many topical formulation types containing water as the major ingredient, including creams, emulsions, gels, ointments, lotions, sprays, solutions, pastes or tinctures. It is preferred, although not required, that the formulation is in the form of an aqueous suspension. Accordingly, the formulation of the invention may contain an agent to stabilize the suspension such as an emulsifier or a viscosity controlling agent. Any emulsifier or viscosity controlling agent that is water-dispersible, suitable for use on epithelial tissue such as skin, and forms an aqueous gel of substantially uniform consistency, is suitable for use in the formulation described herein.

The term **"emulsifier"** as used herein refers to a suitable pharmaceutically acceptable surfactant. Examples of emulsifiers include naturally occurring phospholipids extracted from egg yolk or soybean (e.g., L-oc- lecithin, soybean (or other sources)), synthetic phosphatidyl cholines or purified phosphatidyl cholines from vegetable origin. Preferred emulsifiers include carboxyvinyl polymers, also known as carbomers, such as are sold under the tradename CARBOPOL® (B. F. Goodrich Co., Akron, OH, USA), ETD 2020™, and ULTREZ® (Noveon, Inc., Cleveland, OH, USA). Particularly preferred emulsifying compounds are glyceryl stearate, lanolin, carbomer, camellia sinensis leaf extract, olea europaea fruit oil, ricinus communis seed oil and propylene glycol.

The term **"viscosity controlling agent"** as used herein refers to any agent that is capable of controlling the thickness of the topical formulation described herein. Typically, viscosity controlling agents are capable of decreasing the sedimentation velocity of the dispersed ingredients by maintaining the viscosity of the suspension at a constant level, and so minimize or delay the formation of precipitates to distribute the active ingredients homogeneously in the whole suspension.

According to a preferred embodiment, the viscosity controlling agent comprises one or more viscosity controlling compounds. Specifically, preferred viscosity controlling compounds are betaine, lanolin, propylene glycol or carbomer, or any combination thereof.

If desired, the formulation of the invention may further include additional dermatologically acceptable excipients typically used in formulations and known to those skilled in the art. Such excipients include, for example, humectants, emollients, pH stabilizing agents, preservatives, chelating agents, anti-oxidants, anti-inflammatory agents, anti-bacterial agents, anti-fungal agents, decongestants, antistatic agents, conditioning agents, and any combination thereof.

According to a specific embodiment, the topical formulation further includes one or more skin conditioning agents. As used herein the term **"skin conditioning agent"** refers to any compound that has a cosmetic or therapeutic effect on the skin, hair, or nails. Examples of a skin conditioning agent include lightening agents, darkening agents such as self-tanning agents, anti-acne agents, shine control agents, anti-microbial agents, anti-inflammatory agents, anti-mycotic agents, antiparasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, firming agents, anti-callous agents, and agents for hair, nail, and/or skin conditioning. Preferably, the skin conditioning agent comprises any one or more of the skin conditioning compounds betaine, aloe vera extract, olea europaea fruit oil, carthamus tinctorius seed oil, ricinus communis seed oil, prunus amygdalus dulcis oil or allantoin.

Specifically, the formulation described herein may comprise a pharmaceutically acceptable carrier comprising more than one emulsifying compound, more than one viscosity controlling compound and/or more than one skin conditioning compound or any combination thereof. Specifically, compounds referred to herein may comprise more than one effect, such as for example an emulsifying, viscosity controlling and/or skin conditioning effect.

According to a specific embodiment, the formulation provided herein is suitable for the cosmetic, non-therapeutic use for non-therapeutically improving the complexion of human skin, preferably for non-therapeutically improving the complexion of human facial skin, and/or as non-therapeutic anti-aging composition. Specifically, the formulation provided herein can comprise cosmetic auxiliary substances and additives such as are conventionally used in such formulations. Exemplary auxiliary substances and additives are the pharmaceutically acceptable carriers described herein.

According to a further specific embodiment, the formulation provided herein is suitable for use in the treatment or prevention of one or more diseases or unhealthy conditions of human skin. Specifically, the formulation provided herein may be used for thickening the epidermis and/or for increasing the number of living skin layers, and/or for improving the skin barrier, and/or for improving the organization of the stratum corneum, and/or for decreasing the skin tissue's irritant potential, and/or as anti-oxidative agent(s), and/or for treating or preventing one or more skin diseases in aging.

Specifically, the formulation described herein is used such that it remains for at least 5 minutes, preferably for at least 10 minutes, on the subject's or patient's skin.

Specifically, the formulation is applied to an area of irritated skin, e.g. of skin reddening, inflammatory lesions, or eczema.

According to a specific example, hand eczema can be successfully treated, in particular chronic hand eczema, applying an effective amount to reduce itchiness, dry skin and/or inflammation by at least 10, 20, 30, 40, or 50%. Specifically, hand eczema can be treated with a high response rate of at least 70, 75, 80, or 85%.

The formulation can be applied as a first line treatment, or a second line treatment following treatment resistance.

Specifically, the formulation is used to treat a patient who suffers from treatment-resistant chronic hand eczema, in particular those, who did not respond to corticosteroid treatment.

The formulation provided herein can be produced by conventional processes known to the person skilled in the art. Specifically, the formulation provided herein is produced by incorporation of the components of the composition described herein in a pharmaceutically acceptable formulation comprising pharmaceutically acceptable carriers. Specifically, the formulation provided herein is produced by formulation of the composition described herein with pharmaceutically acceptable carriers, preferably in such a way that it can be applied topically to a subject's skin.

The examples described herein are illustrative of the present invention and are not intended to be limitations thereon. Many modifications and variations may be made to the techniques described and illustrated herein without departing from the spirit and scope of the invention. Accordingly, it should be understood that the examples are illustrative only and are not limiting upon the scope of the invention.

### EXAMPLES

### Example 1: Production of plant extracts

4 g dried and crushed leaves and stems of urtica dioica were transferred into a heat-stable glass bottle with screw cap (200 ml or 500 ml from e.g. Schott) and 96 ml RO or HQ water were added. The contents were mixed in the bottle, the screw cap opened at least one turn and the bottle was placed in a suitable autoclave. After reaching 140°C, the temperature was maintained for 30 minutes and then allowed to cool to 95-98°C before opening the autoclave. The contents of the bottle were filtered through a paper filter as quickly as possible, at least at 85°C. The filter was then removed from the autoclave and the temperature maintained at 95-98°C before opening. In the case of large quantities (containers), the remaining quantity can be kept warm in a preheated water bath (90-95°C). The filtered extract is either processed immediately (e.g. to a cream) or heated again in a water bath to 90°C and stored in the closed container.

The extract of punica granatum is commercially available and e.g. obtained from Aceto Corporation, Carrubba, Inc., Ceramiracle, Inc., Naturex S.A., Sabinsa Corporation, or Xian Yuensun Biological Technology Co.,Ltd.

The extract of hibiscus sabdariffa is commercially available and e.g. obtained from Advanced Biotech, Carrubba, Inc., Grau Aromatics GmbH&Co KG, Naturex S.A., SRS Aromatics Ltd., or Symrise AG.

The extract of camellia sinensis is commercially available and e.g. obtained from Advanced Biotech, Carrubba, Inc., Grau Aromatics GmbH&Co KG, Naturex S.A., SRS Aromatics Ltd., or Symrise AG.

### Example 2: Preparation of a topical formulation, CX101

The extracts obtained according to Example 1 are mixed to create a base substance, which, if all four extracts (urtica dioica leaf extract, camellia sinensis leaf extract, punica granatum peel extract and hibiscus sabdariffa flower extract) are included contains the following:

**Table 1: Composition of the base substance of the topical formulation CX101. The table shows the exposure per cm² skin to each g of ingredient [Exposure] and the systemic exposure dose [SED].**

| Mass % | INCI | SED (g/kg BW/day) | Exposure (g/cm2) |
|---|---|---|---|
| 7.90E+01 | Water | 6.24E-02 | 2.90E-03 |
| 6.06E-01 | Urtica Dioica Leaf Extract | 3.67E-06 | 2.23E-05 |
| 3.03E-01 | Camellia Sinensis Leaf Extract | 9.18E-07 | 1.11E-05 |
| 4.04E-01 | Punica Granatum Peel Extract | 1.63E-06 | 1.48E-05 |
| 4.04E-01 | Hibiscus Sabdariffa Flower Extract | 1.63E-06 | 1.48E-05 |
| 5.10E-01 | Potassium Hydroxide | 2.60E-06 | 1.87E-05 |
| 5.05E+00 | Glyceryl Stearate | 2.55E-04 | 1.85E-04 |
| 3.03E+00 | Betaine | 9.18E-05 | 1.11E-04 |
| 2.53E+00 | Aloe vera callus extract | 6.38E-05 | 9.27E-05 |
| 1.26E+00 | Olea Europaea Fruit Oil | 1.59E-05 | 4.64E-05 |
| 1.26E+00 | Carthamus Tinctorius Seed Oil | 1.59E-05 | 4.64E-05 |
| 1.26E+00 | Ricinus Communis Seed Oil | 1.59E-05 | 4.64E-05 |
| 1.26E+00 | Prunus Amygdalus Dulcis (Sweet Almond) Oil | 1.59E-05 | 4.64E-05 |
| 1.01E+00 | Allantoin | 1.02E-05 | 3.71E-05 |
| 5.05E-01 | Lanolin | 2.55E-06 | 1.85E-05 |
| 6.06E-01 | Propylene Glycol | 3.67E-06 | 2.23E-05 |
| 6.06E-01 | Carbomer | 3.67E-06 | 2.23E-05 |
| 2.02E-01 | Diazoliniyl Urea | 4.08E-07 | 7.42E-06 |
| 2.02E-01 | Methylparaben | 4.08E-07 | 7.42E-06 |

For quality assurance and to determine the ingredients of the base substance a certain quantity (for exemplary purpose hereinafter 100 g) is weighed in and adjusted to pH 2.5 to 3 with 1 N HCI under continuous stirring. The demixed emulsion is then centrifuged at 20°C for 15 min at a minimum weight of 15,000 g. The fat layer (glycerine monostearate) originating from the cream is pierced with a pointed object (e.g. with a clean pipette tip) and the liquid phase underneath is sucked off and filtered through a paper filter. The filtrate is adjusted again to pH 6.6 to 6.8 with 1 N caustic solution and, following centrifugation at 15.000g for 15 minutes, analyzed using HPLC.

To produce the topical formulation CX101, tretinoin is added to the base substance and dissolved using a solvent present in the base substance at higher temperature (above 50°C) and mixed into a cream. In this example, propylene glycol was used as solvent. The amount of tretinoin to be present in the final product shall be between 0.05% and 0.3%.

The amount of plant extracts and vitamin A contained in CX 101 are as follows:
- Urtica Dioica Leaf Extract: 0.606 % (w/w)
- Camellia Sinensis Leaf Extract: 0.303 % (w/w)
- Punica Granatum Peel Extract: 0.404 % (w/w)
- Hibiscus Sabdariffa Flower Extract: 0.404 % (w/w)
- Vitamin A (Tretinoin): 0,1% (w/w), (1mg/10g)

### Example 3: Use of the topical formulation for the treatment of hand eczema

The study was designed as a 21 days observation study. Subjects were instructed to apply CX 101 topically 2-3 times a day. So far 12 subjects have used CX 101 and the results have been very encouraging. Current topical treatments and also other 2nd line treatments tend to suppress the inflammation and the disease. CX 101 doesn't suppress, but rather acts anti-inflammatory and is intended to stimulate normal cell growth. A significant proportion of the subjects included in the study were long-term sufferers with a long history of unsuccessful treatments. They could be considered treatment-resistant long-term chronic sufferers. As such, a highly complex and complicated cohort.

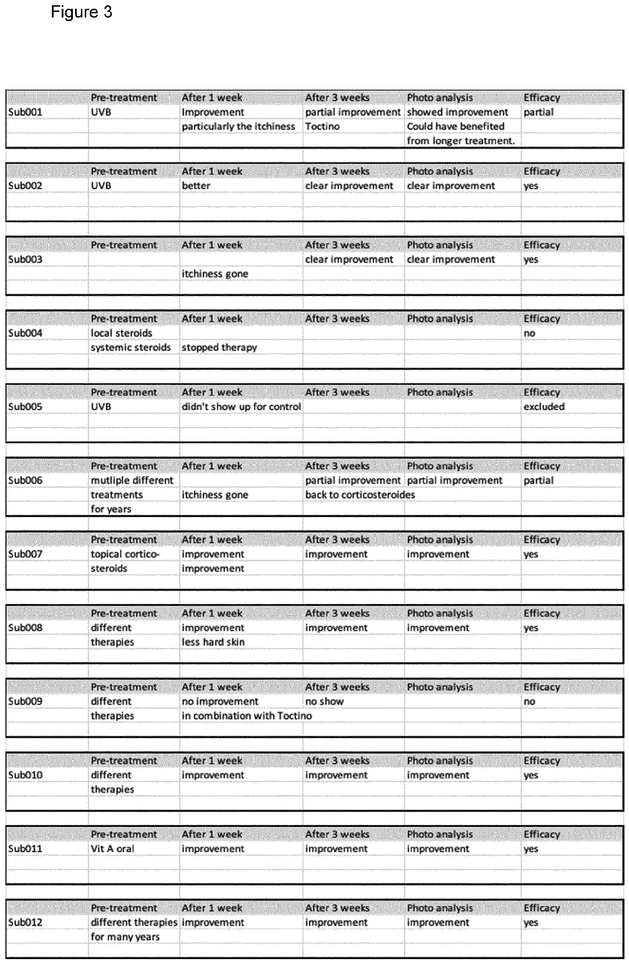

From the initial observations, CX 101 leads to a positive treatment result in 9 out of 12 subjects (Fig. 3):
- 7 subjects showed strong improvements after 3 weeks
- 2 subjects showed an improvement after 3 weeks
- 2 subjects stopped the treatment due to the break-out (which could likely be due to the suppressive nature of the treatment used before CX 101)
- 1 subject dropped-out (no show)

The tests indicate a high efficacy with a response rate of almost 82% as well as a high tolerability. Whereby the response rate was measured by subjects with visible improvements. The rate being calculated as 9 subjects with improvements / 11 subjects followed-up (9/11 = 81,82%).

A significant reduction of itchiness was described by most subjects. After 3 weeks of use, significant reduction in the inflammation underlying the chronic hand eczema and reduction in the chronic hand eczema itself could be observed. 9 (nine) of 11 (eleven) subjects that used the product for at least 3 weeks showed a positive reaction after 3 weeks.

## Claims

1. A topical formulation comprising a composition comprising a water extract of plant material of urtica dioica and a vitamin A compound, and a pharmaceutically acceptable carrier.

2. The formulation of claim 1, wherein the composition further comprises at least one extract of plant material of any one or more of punica granatum, hibiscus sabdariffa, and camellia sinensis.

3. The formulation of claim 1 or 2, wherein the plant material is peel, leaf, stem and/or flower.

4. The formulation of any one of claims 1 to 3, wherein the plant material of urtica dioica is leaf and/or stem material, preferably extracted using water at a temperature ranging from 120°C to 140°C.

5. The formulation of any one of claims 1 to 4, wherein the composition comprises p-Coumaric acid, coffeoyl malate, coumarin and trans-ferulic acid, preferably wherein a first mixture of p-Coumaric acid and caffeoyl malate and a second mixture of coumarin and trans-ferulic acid are present at a ratio of 0.5-2:1 (w/w), preferably 0.8-1.5:1.

6. The formulation of any one of claims 1 to 5, wherein each extract urtica dioica, punica granatum, hibiscus sabdariffa, or camellia sinensis is comprised at a concentration of 0.1 to 5 % (w dry substance/w), preferably 0.2 to 0.7 % (w dry substance/w).

7. The formulation of any one of claims 1 to 6, wherein the vitamin A compound is vitamin A, a derivative of vitamin A or provitamin A, preferably selected from the group consisting of tretinoin, retinol, retinal, retinoic acid, palmitate, β-carotene, adapalene, tazarotene, lutein, and zeaxanthin, preferably wherein the vitamin A compound is comprised at a concentration of 0.05 to 0.3 % (w/w).

8. The formulation of any one of claims 1 to 7, wherein the pharmaceutically acceptable carrier comprises at least one emulsifier at a concentration of 0.1 to 10 % (w/w), preferably wherein the emulsifier is any one or more of glyceryl stearate, lanolin, carbomer, camellia sinensis leaf extract, olea europaea fruit oil, ricinus communis seed oil or propylene glycol.

9. The formulation of any one of claims 1 to 8, wherein the pharmaceutically acceptable carrier comprises at least one viscosity controlling agent at a concentration of 0.1 to 10 % (w/w), preferably wherein the viscosity controlling agent is any one or more of betaine, lanolin, propylene glycol or carbomer.

10. The formulation of any one of claims 1 to 9, wherein the pharmaceutically acceptable carrier comprises at least one skin conditioning agent at a concentration of 0.1 to 10 % (w/w), preferably wherein the skin conditioning agent is any one or more of betaine, aloe vera extract, punica granatum, hibiscus sabdariffa, camellia sinensis, olea europaea fruit oil, carthamus tinctorius seed oil, ricinus communis seed oil, prunus amygdalus dulcis oil or allantoin.

11. The formulation of any one of claims 1 to 10, which is in the form of a cream, emulsion, gel, ointment, lotion, spray, solution, paste or tincture.

12. A method of producing the formulation of any one of claims 1 to 11, comprising providing said extract(s) and said vitamin A compound, and formulating with said pharmaceutically acceptable carrier.

13. The method of claim 12, comprising the steps of:
i. providing the components of said composition, which are the vitamin A compound, the water extract of urtica dioica, and optionally at least one extract of plant material of any one or more of punica granatum, hibiscus sabdariffa, and camellia sinensis;
ii. mixing said components in an aqueous solution;
iii. adding at least one emulsifier;
iv. and optionally further adding any one or more of viscosity controlling agents, skin conditioning agents, buffering agents, emollients, antimicrobial agents or preservatives,
wherein said steps ii., iii., and iv., are carried out at a temperature of 50-90°C, followed by allowing the mixture to cool under stirring to form the topical formulation.

14. Use of the formulation of any one of claims 1 to 11 in a cosmetic product, preferably selected from the group consisting of a skin cream, emulsion, ointment, gel, lotion, spray, solution, paste and tincture.

15. The formulation of any one of claims 1 to 11, for use in the treatment of skin related diseases or conditions, preferably dermatitis, psoriasis or eczema.
